(19) Europäisches Patentamt
European Patent Office
Office européen des brevets

(11) **EP 3 610 862 A1**

(12) **EUROPEAN PATENT APPLICATION**

(43) Date of publication:
**19.02.2020 Bulletin 2020/08**

(51) Int Cl.:
*A61K 9/28* (2006.01)     *A61K 9/48* (2006.01)

(21) Application number: **18188848.8**

(22) Date of filing: **14.08.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Apillet APS**
**4000 Roskilde (DK)**

(72) Inventors:
• **Petersen, Carsten Lunde**
**4000 Roskilde (DK)**

• **Christensen, Anders**
**4000 Roskilde (DK)**

(74) Representative: **Kjerrumgaard, Lars Bo**
**LBKPATENT APS**
**Cobis Ole Maaløes Vej 3**
**2200 Copenhagen (DK)**

Remarks:
Claims 16-23 are deemed to be abandoned due to non-payment of the claims fees (Rule 45(3) EPC).

(54) **NOVEL ORAL COMPOSITION**

(57)     The present invention relates to a solid oral composition for targeted release in an intestine of a mammal comprising a core and a coating completely surrounding the core, wherein the core comprises an entrapped material to be released in the intestine, and the coating or a part of the coating is adapted to be digested/to self-perforate in the intestine.

Figure 4

Time (min)

Stomach transit time 127 min.

Small intestine transit time 162 min.

**Description**

**Technical field**

The present invention relates to a solid oral composition for targeted release in an intestine of a mammal. Moreover, the present invention is concerned with the solid oral composition for different uses, such as for use as a medicinal product, for use as a nutritional supplement, or for use as a tracing agent. Furthermore, the present invention relates to the use of a pair of components for preparing a coating for a solid oral dosage form.

**Background Art**

Oral delivery of active pharmaceutical ingredients is often the chosen route of delivery since it is easier, more convenient and generally painless, resulting in larger patient adherence relative to other means of delivery.

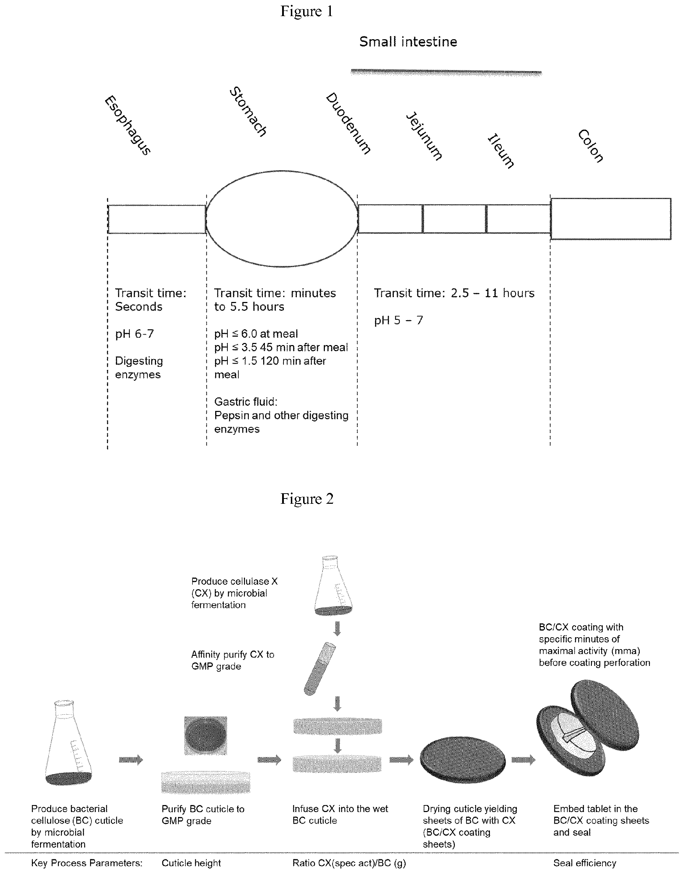

Therapeutically active and other important biological compounds may be administered to patients in a variety of ways, including for example, by the oral route. To administer proteins and peptides, but also sensitive small molecule therapeutics by the oral route is a challenge and has been pursued for many years. Some of the challenges include, but are not limited to, digestive enzymes and the strongly acidic environment of the stomach, components of pancreatic juices and secretions of the biliary system. Because of these challenges an unreliable bioavailability may be the consequence if sensitive active pharmaceutical ingredients are orally delivered. In addition, the compositions and concentrations of the gastro intestinal (GI) components are not uniform but vary within the segments of the GI tract. Some of the challenges for peptides, proteins and other sensitive substances in the GI tract are depicted in figure 1.

One skilled in the art will appreciate that, to increase efficiency and bioavailability, the dosage form should deliver the active agent (entrapped material), and any required excipient, to the part of the GI tract best suited chemically and physically for absorption of that active ingredient. There is a need for enteric coatings that can provide suitable bioavailability of such administered proteins, peptides and small molecules.

**Summary of the invention**

In a first aspect the present invention relates to a solid oral composition for targeted release in an intestine of a mammal comprising a core and a coating completely surrounding the core, wherein the core comprises an entrapped material to be released in the intestine, and the coating or a part of the coating is adapted to be digested/to self-perforate in the intestine.

In one embodiment the intestine is selected from small intestine, large intestine, duodenum, ileum, jejunum, and colon.

In a further embodiment the coating is adapted to resist breakdown from the environment in a stomach of a mammal.

In a still further embodiment the coating prevents the release of the entrapped material in a stomach of a mammal.

In a further embodiment the coating protects the entrapped material in a stomach of a mammal.

In a still further embodiment the coating is adapted to release the entrapped material in an intestine of a mammal.

In a further embodiment the coating comprises a pair of components, in particular the pair of components are in contact with each other in the coating. When the coating consists of two components such components are in contact with each other, with no intermediate layer, to be ready to initiate the reaction in the intestine as explained in detail herein.

In a still further embodiment the first component is resistant to an environment in the stomach of a mammal and the second component enzymatically digest the first component when subjected to the more basic environment of the small intestine compared to the more acidic environment of the stomach.

In a further embodiment the digesting activity of the second component is inhibited in the environment in the stomach of a mammal.

In a still further embodiment the mammal is selected from animals with little or no ability to digest dietary fibers like cellulose, such as a human, a monkey, a pig, a dog, a human ape, a rodent and a cat.

In a further embodiment the entrapped material is a protein, enzyme, polypeptide, oligopeptide, peptide, deoxyribonucleic acid (DNA), ribonucleic acid (RNA), a small organic molecule of less than 900 Da, or a pro-drug of any one of these materials, is a nutritional supplement, or a microbial culture, or a microbial additive, or a vaccine. Further embodiments of the entrapped material are selected from:

a) Proteins, Human growth hormone (hGH), Calcitonin, Insulin, GLP-1 analogues, GLP-1,
b) Peptides, Octreotide,
c) Oral vaccines, Oral cholera vaccine, Mycoplasma hyopneumoniae oral vaccine, Live bacterial cells as attenuated vaccines,

d) Small organic molecules 200<MW<900 g/mol, Desmopressin, Vasopressin, Cyclo-sporine, Ranitidine, Diclofenac, Ketoprofen, Amifostine, Omeprazole, Gemcitabine, Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Lobenzarit, Genistein, Verapamil, Terbinafine, Lornoxicam, Clotrimazole.

In a still further embodiment the first component and the second component are mixed.

In a further embodiment the first component and the second component are in different layers with the first component being the outer layer and the second component the inner layer around the core.

In a still further embodiment the pair of the first and second component is selected from dietary fibers and enzymes that digest dietary fibers like cellulose and cellulase, pectin and pectinolytic enzymes, hemicellulose and hemicellulase, lignin and lignin degrading enzymes, fructan and fructan degrading enzymes, and lipid and lipases.

In a further embodiment the pair of the first and second component is selected from structurally ordered cellulose I, such as bacterial cellulose or derivatives thereof and a cellulase, such as cellulase (EC 3.2.1.4) (endocellulase) and cellubiase (EC 3.2.1.21) (beta-glucosidase) and 1,4-beta-cellobiosidase (EC 3.2.1.91) (Exocellulase).

In a still further embodiment the second component is enzymatically active and digests the first component in the environment in the intestine of a mammal. Typically, the second component has maximum enzymatic activity in the range pH 3-12, such as from pH 3.5 to 9.5, or from pH 3.0 to 9.0, or from pH 4.0 to 9.0.

In a further embodiment the composition is a tablet or a capsule or other type of solid oral dosage form.

In a second aspect the present invention relates to a composition of the first aspect or any one of the above embodiments, for use as a medicinal product. In one embodiment the medicinal product is in the form of a microbial culture.

In a third aspect the present invention relates to a composition of the first aspect or any one of the above embodiments for use as a nutritional supplement.

In a fourth aspect the present invention relates to a composition of the first aspect or any one of the above embodiments for use as a tracing agent.

In a further aspect the present invention relates to use of a pair of components for preparing a coating for a solid oral dosage form where the coating is degraded when subjected to a pH change from a lower to a higher pH, wherein the digestion of the first component by the second component is inhibited at the lower pH and the second component digest the first component when subjected to the higher pH.

**Description of the invention**

The present invention relates to a solid oral composition for targeted release in an intestine of a mammal. The solid oral composition may be any form known to the skilled person and in particular is a tablet or a capsule, to be administered via the oral route to a mammal. In one embodiment the oral composition is a tablet. In another embodiment the oral composition is a capsule. The mammal can be any mammal, such as a human.

The term "mammal" as used herein means animals, such as without limitation humans, dog, cat, pig, monkey, human apes, rodents. The term "mammal with little or no ability to digest cellulose" means human, dog, cat, pig, monkey, human apes, rodents and other animals with little or no ability to digest cellulose. Mammals, such as cows, cattle, horse, goat, sheep and deer, that natively have a digestion system with ability to digest cellulose polymers do not benefit from the present invention when the coating contains celluloses.

The solid oral composition for targeted release in the intestine of a mammal, such as a human, refers to the design of the solid composition as explained in detail below, which design allows accurate sensing of when the composition reaches the intestine, which is the target, hence targeted release.

The solid composition comprises a core, which core may contain a solution, suspension or be a solid core, provided the core itself does not have any influence on the stability of the coating. The core is surrounded by a coating which defines the outer solid layer, hence the overall composition is referred to as a solid composition. In the core a material is contained and entrapped, which material is to be released in the intestine upon the coating or part of the coating being dissolved or destroyed. The entrapped material can be any material suitable for release in the intestine, such as without limitation, a drug, a nutrient, a supplement, a microbial organism, a vaccine, a radio transmitter, a device for measuring parameters in the intestine, etc. The coating or a part of the coating is adapted to be digested in the intestine, which means that the coating may consist of different coatings, such as a part to be digested and release the entrapped material and another part which remains intact, or the whole coating is to be digested or perforated in the intestine and release the entrapped material. The intestine comprises different elements, such as the small intestine, large intestine which consists of different elements duodenum, ileum, jejunum, and colon. It is intended that each of these elements can be subject to an embodiment in combination with the aspects and embodiments of the present invention.

Preferably, the coating is adapted to resist breakdown from the environment in a stomach of a mammal, so that no release of the entrapped material takes place in the stomach. The coating may in itself be made of an element that resists the stomach environment, such as low pH of a human stomach, but dissolves or perforates in the intestine, or as explained detailed herein may be composed of two or more elements where one enzymatically digests the other in the intestine, but not in the stomach. Thus, the coating prevents the release of the entrapped material in a stomach of a mammal, and/or the coating protects the entrapped material in a stomach of a mammal. When stated that the coating is adapted to release the entrapped material in an intestine of a mammal, it refers to the composition of the coating which composition will be disrupted by enzymatical digestion in the intestine. Such a coating may be composed of one or more components, and in accordance with a preferred embodiment of the present invention the coating comprises a pair of components. The pair of components means two components and such components are different in that one must digest the other in order to make this a coating as used in accordance with the invention, wherein the coating or a part of the coating is adapted to be digested in the intestine.

The first component is resistant to the environment in the stomach of a mammal and the second component digest the first component when subjected to the more basic environment of the intestine compared to the more acidic environment of the stomach. In this respect the mammal is preferably selected from animals with little or no ability to digest cellulose, such as a human, a monkey, a pig, a dog, a human ape, a rodent and a cat. In a preferred embodiment the mammal is a human. The reference to first and second is only to indicate that the two components are different but does in no way refer to the way or any order of making the coating. Preferably, the second component digests the first component by having a digesting activity that is inhibited in the environment in the stomach of the mammal. The second component if not inhibited in the environment in the stomach may become a less workable solution although this could be compensated for by adding a further coating covering the solid oral composition of the present invention.

One method of making a coating for use in accordance with the present invention is the preparation of the pair of components. The first component of the coating composition for use in the present invention can for instance be produced by first producing a bacterial cellulose (BC) cuticle by microbial fermentation, secondly purify the BC. This procedure may include an alkaline treatment step. Then the second component, such as a cellulase can be produced by microbial fermentation, chemical synthesis or other means, followed by a purification of this cellulase, for instance by purifying by affinity chromatography. Hereafter, the cellulase is made in a fluid formulation and infused into the BC cuticle (for instance at a pH or at other conditions where the cellulase is inactive). Dry the cuticle to obtain sheets of BC impermeable to molecules of MW > 200 Da containing the cellulase (CX). Embed core (comprising the entrapped material to be released in the intestine) in the BC/CX sheets and seal with an appropriate component. An outline of a coating production process is shown in figure 2.

The entrapped material can be anything which is suitable for release in the intestine, such as compounds for use as medicine.

In a further embodiment the entrapped material is a drug. In a still further embodiment the entrapped material is a nutritional supplement. In a further embodiment the entrapped material is a microbial culture. In a still further embodiment the entrapped material is a microbial additive. In a further embodiment the entrapped material is a vaccine.

Further embodiments of the drug are selected from one or more of a protein, an enzyme, a polypeptide, an oligopeptide, a peptide, a deoxyribonucleic acid (DNA), ribonucleic acid (RNA), a small organic molecule of less than 900 Da, or a pro-drug of any one of these materials, and all of these are considered individual embodiments and may be subject to one or more claims in respect of any one of the aspects and embodiments of the aspects described herein.

Further embodiments of the entrapped material are selected from one or more of:

a) Proteins, Human growth hormone (hGH), Calcitonin, Insulin, GLP-1 analogues, GLP-1,
b) Peptides, Octreotide,
c) Oral vaccines, oral cholera vaccine, *Mycoplasma hyopneumoniae* oral vaccine, live bacterial cells as attenuated vaccines,
d) Small organic molecules 200<MW<900 g/mol, Desmopressin, Vasopressin, Cyclo-sporine, Ranitidine, Diclofenac, Ketoprofen, Amifostine, Omeprazole, Gemcitabine,

Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Loben-zarit, Genistein, Verapamil, Terbinafine, Lornoxicam, Clotrimazole. Each of the compounds or vaccines are considered individual embodiments and may be subject to one or more claims in respect of any one of the aspects and embodiments of the aspects described herein.

The pair of components may be mixed or may be in separate layers. Other ways of making a coating consisting of two components are contemplated by the invention as long as the two components are in contact with each other. For instance, the first component and the second component are mixed. Alternatively, the first component and the second component are in different layers, such as two layers wherein the first component being the outer layer and the second component the inner layer around the core.

In a still further embodiment the pair of the first and second component is dietary fibers and enzymes that digest dietary fibers. In a further embodiment the pair of the first and second component is cellulose and cellulase. In a further embodiment the pair of the first and second component is pectin and pectinolytic enzymes. In a still further embodiment the pair of the first and second component is hemicellulose and hemicellulase. In a further embodiment the pair of the first and second component is lignin and lignin degrading enzymes. In a still further embodiment the pair of the first and second component is fructan and fructan degrading enzymes. In a further embodiment the pair of the first and second component is lipid and lipases.

When the pair of the first and second component is cellulose and cellulase, preferably, the first and second component is selected from structurally ordered cellulose I and a cellulase. The most abundant type of native cellulose found in nature is called cellulose I[6, 7, 8]. The structurally ordered cellulose I is preferably, a bacterial cellulose or derivatives thereof and the cellulase is preferably selected from the group consisting of cellulase (EC 3.2.1.4) (endocellulase), cellubiase (EC 3.2.1.21) (beta-glucosidase) and 1,4-beta-cellobiosidase (EC 3.2.1.91) (Exocellulase).

The term "structurally ordered cellulose I" or "native cellulose" as used interchangeable herein refers to an unbranched polymer containing any number of eight or more D-glucose units, linked by $\beta$-1,4 glycosidic bonds.

In a still further embodiment the second component is enzymatically active and digests the first component in the environment in the intestine of a mammal. In particular, the second component has maximum enzymatic activity in the range pH 3-12, such as pH 3.5 to 9.5, or 3.0 to 9.0, or 4.0 to 9.0.

Furthermore, the present invention relates to a solid oral composition for targeted release in an intestine of a mammal comprising a core and a coating completely surrounding the core, wherein the core comprises an entrapped material to be released in the intestine, and the coating or a part of the coating is adapted to be digested in the intestine, for use as a medicinal product. Each of the above described embodiments in relation to the first aspect also applies to this particular use aspect.

Furthermore, the present invention relates to a solid oral composition for targeted release in an intestine of a mammal comprising a core and a coating completely surrounding the core, wherein the core comprises an entrapped material to be released in the intestine, and the coating or a part of the coating is adapted to be digested in the intestine, for use as a nutritional supplement. Each of the above described embodiments in relation to the first aspect also applies to this particular use aspect.

Furthermore, the present invention relates to a solid oral composition for targeted release in an intestine of a mammal comprising a core and a coating completely surrounding the core, wherein the core comprises an entrapped material to be released in the intestine, and the coating or a part of the coating is adapted to be digested in the intestine, for use as a tracing agent. Each of the above described embodiments in relation to the first aspect also applies to this particular use aspect.

In a further aspect the present invention relates to use of a pair of components for preparing a coating for a solid oral dosage form where the coating is degraded when subjected to a pH change from a lower to a higher pH, wherein the first component is adapted to resist digestion at the lower pH and the second component digest the first component when subjected to the higher pH. The coating may be used to surround the solid oral dosage form or cover a part of the dosage form as the case may be. Typically, the solid oral dosage form is a tablet, a capsule or granulates. Typically, the pH change is from 1.0 - 3.5 in the stomach to 5.5- 7.5 in the intestine. The creation of the coating according to this further aspect which coating can be stored for later use is also contemplated by this aspect of the invention. The coating can be used in setting up test systems to verify a suitable pair of components and amounts and ratios to be used.

The above embodiments should be seen as referring to any one of the aspects (such as 'composition, 'pharmaceutical composition', 'composition for use as a medicinal product, or 'compound for use in a method') described herein as well as any one of the embodiments described herein unless it is specified that an embodiment relates to a certain aspect or aspects of the present invention.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

The term "and/or" as used herein is intended to mean both alternatives as well as each of the alternatives individually. For instance, expression "xxx and/or yyy" means "the xxx and yyy; the xxx; or the yyy", all three alternatives are subject to individual embodiments.

Recitation of ranges of values herein are merely intended to serve as a shorthand method of referring individually to each separate value falling within the range, unless other-wise indicated herein, and each separate value is incorporated into the specification as if it were individually recited herein. Unless otherwise stated, all exact values provided herein are representative of corresponding approximate values (*e.g.*, all exact exemplary values provided with respect to a particular factor or measurement can be considered to also provide a corresponding approximate measurement, modified by "about," where appropriate).

All methods described herein can be performed in any suitable order unless other-wise indicated herein or otherwise clearly contradicted by context.

The use of any and all examples, or exemplary language (*e.g.*, "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (*e.g.,* a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

## Experimental procedures

**Abbreviations**

| | |
|---|---|
| BC | Bacterial cellulose |
| CCH | Cumulated cellulose hydrolysis |
| mma | Minutes of maximal activity |
| %ma | Percentage of maximal activity |
| pHd | pH duodenum |
| pHs | pH stomach |

## Methods and results

Physiological parameters were extracted for gastro intestinal pH and passage times in humans from literature. From these parameters, a semi-physiological model of the pH in the GI tract was synthesized and validated against literature data. Further, we extracted biochemical parameters for cellulases characterized by having activity maxima around neutral pH and strongly inhibited activity at acidic pH from the Brenda bioinformatics database [9] and from literature [10, 11, 12]. From these data a semi-biochemical model of the pH dependency of cellulase activity was synthesized and validated against literature data. By combining these two models a semi-physiological model of cumulated cellulase activity was synthesized and used to perform simulations of cellulase passing through the human GI tract.

Results: The two synthesized models predicted the literature data well. Simulations showed that the cellulase activity is strongly inhibited as the cellulase passes through the stomach and that the cellulase becomes active when entering the small intestine. Further, simulations showed only a small amount of cumulated activity (cumulated cellulose breakdown) takes place during the passage of the stomach compared to the cumulated activity (cumulated cellulose breakdown) that takes place during the passage of the small intestine, even at physiologically highly prolonged stomach passage times and physiologically very short small intestine passage time. This shows there is a robust design space for a cellulose-cellulase based coating which protects the entrapped material during the passage of the stomach and which perforates in the small intestine.

The entero coating presented here is a method which robustly protects an entrapped material during the passage of the stomach and which robustly releases the material in the small intestine through a self-perforating mechanism.

**Results**

Cellulases with a maximal activity in the pH range 5.5 - 8.0 and with activity at most 25 % of maximal activity (%ma) in acidic environments (pH < 4.0) were identified in the Brenda bioinformatics database and in literature [9, 10, 11, 12] and biochemical characterization data from these cellulases were used to synthesize a model ($A_1$(pH)) predicting the relative cellulase activity as a function of pH.

$$A_1(pH) = 100 * exp\left(-\left(\frac{|pH-6.5|}{2}\right)^3\right) \%ma$$

The synthesised activity function $A_1$(pH) models a cellulase with maximal activity at pH 6.5.

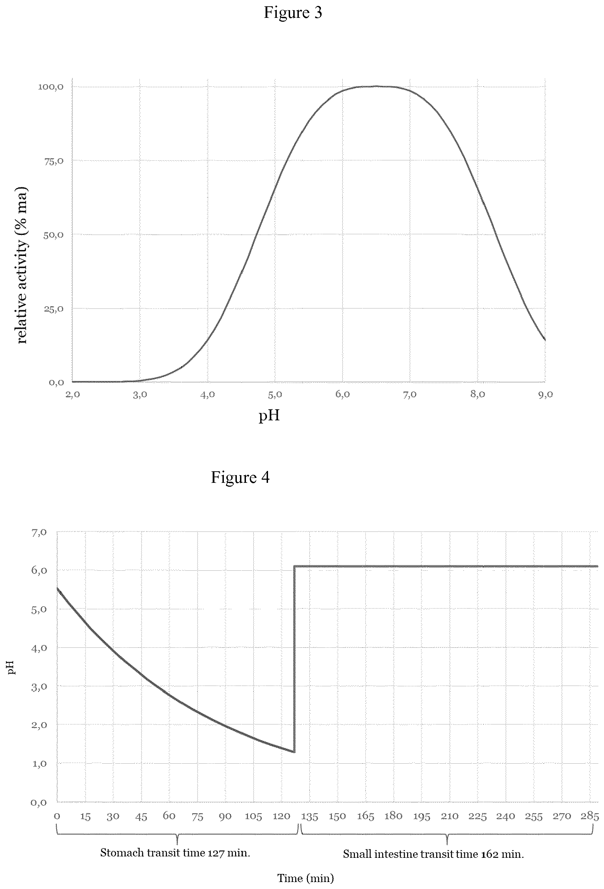

A graphic representation of the relative activity as a function of pH is shown in figure 3. The model was validated against literature data by overlaying the synthesized $A_1$(pH) activity function with the activity data from cellulases identified in the literature [10, 11, 12].

A model for pH(t) in the human gastro intestinal system was synthesized by combining physiological data from the literature [1, 2].

The model for the variation of pH in the stomach (*pHs(t)*) of healthy young men and women with time after meal ingression was extracted from the study [1].

$$pH_S(t) = 0.5 + \exp(-0.016 * t + 1.7)$$

The model with constant pH = 6.1 for the pH in the small intestine is based on the study [2]. Denoting by $T_S$ the stomach transit time and by $T_I$ the intestinal transit time we obtain a model pH(t) predicting the human gastro intestinal pH environment surrounding a capsule/ an entrapped material as it passes through the intestine. Figure 1 shows a graphic representation of the synthesized model $A_1$(pH) predicting the relative cellulase activity as a function of pH.

$$pH(t) = \begin{cases} 0.5 + \exp(-0.016 * t + 1.7), & 0 \leq t \leq T_S, \\ 6.1, & T_S \leq t \leq T_S + T_I. \end{cases}$$

The model *pH(t)* was plotted on top of human stomach and intestinal physiological data and predicts well the data on gastro intestinal pH in healthy humans found in the literature [1,2]. Figure 4 shows the graphic representation of the model predicting the gastro intestinal pH(t) in humans with a stomach transition time $T_S$ of 127 min and a short small intestine transit time $T_I$ of 162 min.

By combining the model ($A_1$(pH)) predicting the relative cellulase activity as a function of pH with the model predicting the gastro intestinal pH(t) in humans, a model of relative cellulase activity in the GI tract was obtained by composing the two functions:

$A_1$(pH(t))

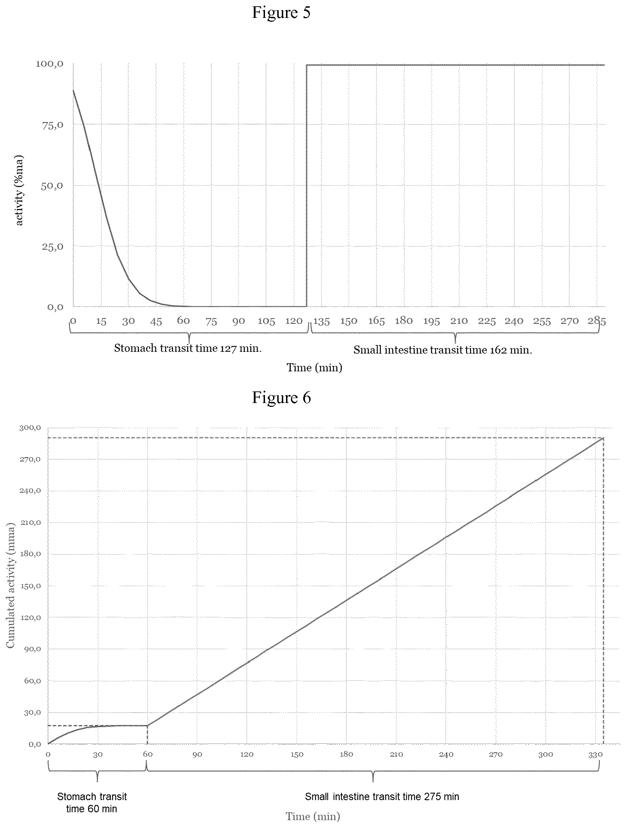

Where $A_1$(*pH*) is the cellulase activity function and *pH(t)* is the human gastro intestinal pH function. Figure 5 shows a graphic representation of the model of relative cellulase activity in the gastro intestinal tract. The relative cellulase activity in the GI tract with a stomach transition time $T_S$ of 127 min and a small intestine transit time $T_I$ of 162 min is shown.

A model predicting the cumulated activity in the self-perforating material, where the first component is cellulose, and the second component is a cellulase, is obtained by integrating the relative activity function $A_1$(*pH(t)*)/100 with respect to time. A graphic representation of the cumulated activity in the self-perforating material with a median stomach transition time $T_S$ of 60 min and a median small intestine transit time $T_I$ of 275 min is shown in figure 6.

For the calculations of cumulated activity, a relative unit minutes of maximal activity (mma) is used. The mma unit describes the cumulated cellulase activity as equivalents of reaction minutes when the reaction is performed at optimal conditions. e.g. at pH 6.5, the optimal reaction pH, in the case of the model cellulase described in $A_1$(pH).

- The basic unit 1 mma is the enzymatic cellulase turnover of β-1, 4-glycosidic bonds at maximal enzymatic activity for one minute.
- The mma unit is related to a specific number of active sites e.g. amount of cellulase and a specific number of 1-4 glycosidic bounds, e.g. amount of cellulose
- The mma unit is specific to individual cellulases and to individual cellulose substrates

The mma unit can be used to compare the cumulated cellulase activity under different reaction conditions, for example reaction time, temperature, pH, cofactor concentration and inhibitor concentrations. The mma unit is convenient for product design and may be used as a production specification to design a material which self-perforate within a certain range of cumulated cellulase activity.

**Example 1**

The model of the performance of the self-perforating material in the gastro-intestinal system $A_1(pH(t))/100$ was used to study the activity cumulating in the self-perforating material when passaging the human GI tract under different physiological conditions. Five studies were made to explore, under different stomach emptying times and small intestine transition times, the properties of the self-perforating material, where the first component is cellulose, and the second component is a cellulase with the activity function $A_1(pH)$.

*A stomach transition time of 60 min and a small intestine transit time of 275 min*

A typical median stomach emptying time was found in the literature to be 60 min [3,4,5] and a typical median intestinal transition time was found to be 275 min in the literature [3,4,5]. A graphic representation of the cumulated cellulose hydrolysis predicted by the model using these stomach-emptying and small intestine transition times is shown in figure 6. Figure 2 shows the model predicting the cumulated activity in the self-perforating material in the gastro-intestinal system. Input parameters: a stomach transition time $T_S$ of 60 min and a small intestine transit time $T_I$ of 275 min.
The model of the cumulated activity in the GI tract shows that a self-perforating material designed to withstand a cumulated activity of more than 17 mma will protect the entrapped material from the environment in the stomach with a stomach emptying time of up to 60 min. In addition, a self-perforating material designed to be digested with a cumulated activity of less than 290 mma will release the entrapped material in the human small intestine given a small intestine transit time of 275 min. Thus, a self-perforating material designed to become digested by a cumulated activity in the range 17 to 290 mma will release the entrapped material with typical median stomach and small intestinal transit times.

*A stomach transition time of 5 min and a small intestine transit time of 162 min*

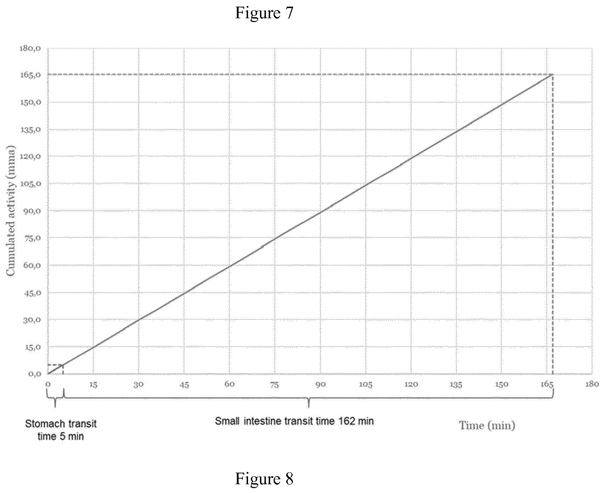

A typical short stomach emptying time was found in the literature to be 5 min [3,4,5] and a typical short intestinal transition time was found to be 162 min in the literature [3,4,5]. A graphic representation of the cumulated cellulose hydrolysis predicted by the model using these stomach-emptying and small intestine transition times is shown in figure 7. Figure 3 shows the model predicting cumulated activity in the self-perforating material in the gastro-intestinal system. Input parameters: a stomach transition time $T_S$ of 5 min and a small intestine transit time $T_I$ of 162 min.
The model of the cumulated activity in the GI tract shows that a self-perforating material designed to withstand a cumulated activity of more than 5 mma will protect the entrapped material from the environment in the human stomach with a stomach emptying time of up to 5 min. In addition, when designed to be digested with a cumulated activity of less than 165 mma it will release the entrapped material in the human small intestine given a small intestine transit time of 162 min. Thus, a self-perforating material designed to become digested by a cumulated activity in the range 5 to 165 mma will release the entrapped material with typical short stomach and small intestinal transit times.

*A stomach transition time of 337 min and a small intestine transit time of 162 min*

A typical long stomach emptying time was found in the literature to be 337 min [3,4,5] and a typical short intestinal transition time was found to be 162 min in the literature [3,4,5]. A graphic representation of the cumulated cellulose hydrolysis predicted by the model using these stomach-emptying and small intestine transition times is shown in figure 8. Figure 8 shows the model predicting the cumulated activity in the self-perforating material in the gastro-intestinal system using input parameters: stomach transition time $T_S$ of 337 min; small intestine transit time $T_I$ of 162 min.
The model of the cumulated activity in the GI tract shows that a self-perforating material designed to withstand a cumulated activity of more than 17 mma will protect the entrapped material from the environment in the human stomach with a stomach emptying time of up to 337 min. In addition, if designed to be digested with a cumulated activity of less than 178 mma the entrapped material will be released in the human small intestine given a small intestine transit time of 162 min. Thus, a self-perforating material designed to become digested by a cumulated activity in the range 17 to 178 mma will release the entrapped material with typical long stomach and short small intestinal transit times.

*A stomach transition time of 5 min and a small intestine transit time of 669 min*

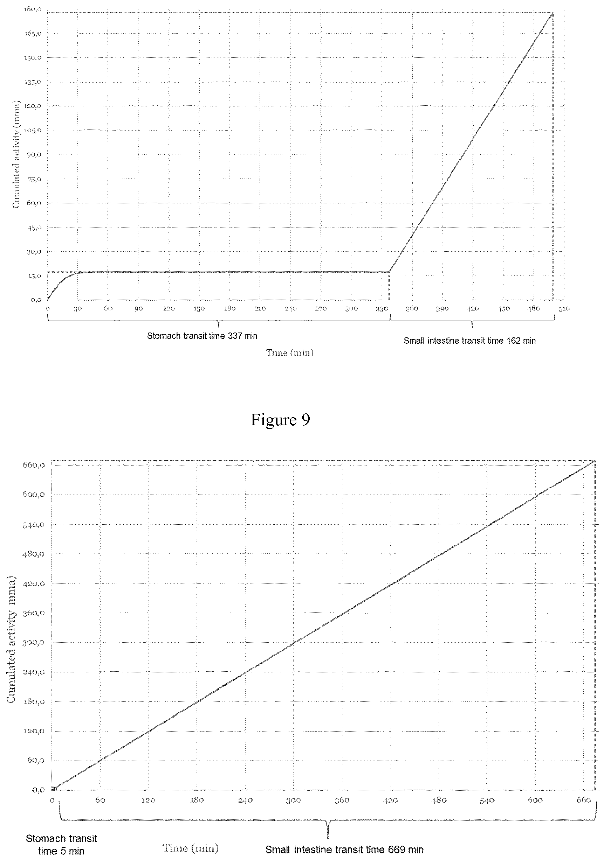

A typical short stomach emptying time was found in the literature to be 5 min [3,4,5] and a typical long intestinal transition time was found to be 669 min in the literature [3,4,5]. A graphic representation of the cumulated cellulose hydrolysis predicted by the model using these stomach-emptying and small intestine transition times is shown in figure 9. Figure 9 shows the model predicting the cumulated activity in the self-perforating material in the gastro-intestinal system. Input parameters: a stomach transition time $T_S$ of 5 min and a small intestine transit time $T_I$ of 669 min.

The model of the cumulated activity in the GI tract shows that a self-perforating material designed to withstand a cumulated activity of more than 5 mma will protect the entrapped material from the environment in the human stomach with a stomach emptying time of up to 5 min. In addition, a self-perforating material designed to be digested with a cumulated activity of less than 668 mma will release the entrapped material in the human small intestine given a small intestine transit time of 669 min. Thus, a self-perforating material designed to become digested by a cumulated activity in the range 5 to 668 mma will release the entrapped material with typical long stomach and short small intestinal transit times.

*A stomach transition time of 337 min and a small intestine transit time of 669 min*

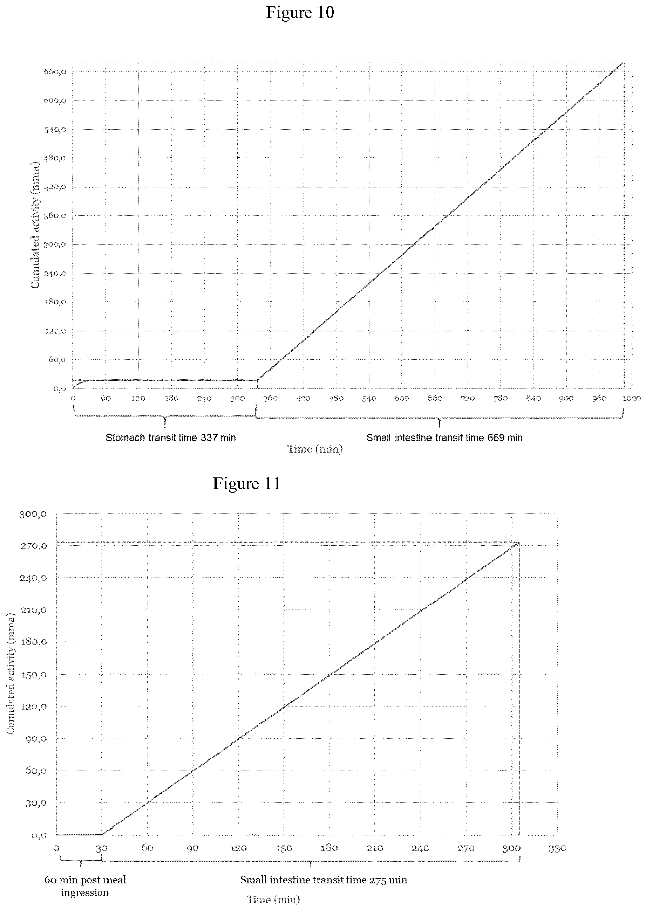

A typical long stomach emptying time was found in the literature to be 337 min [3,4,5] and a typical long intestinal transition time was found to be 669 min in the literature [3,4,5]. A graphic representation of the cumulated cellulose hydrolysis predicted by the model using these stomach-emptying and small intestine transition times is shown in figure 10. Figure 10 shows the model predicting the cumulated activity in the self-perforating material in the gastro-intestinal system. Input parameters: a stomach transition time $T_S$ of 337 min and a small intestine transit time $T_I$ of 669 min.

The model of the cumulated activity in the GI tract shows that a self-perforating material designed to withstand a cumulated activity of more than 17 mma will protect the entrapped material from the environment in the human stomach with a stomach emptying time of up to 337 min. In addition, a self-perforating material designed to be digested with a cumulated activity of less than 681 mma will release the entrapped material in the human small intestine given a small intestine transit time of 669 min. Thus, a self-perforating material designed to become digested by a cumulated activity in the range 17 to 681 mma will release the entrapped material with typical long stomach and long small intestinal transit times.

In the five studies in example 1 all combinations of typical short and long stomach emptying times and typical short and long intestinal transition times were studied. The self-perforating material, where the first component is cellulose, and the second component is a cellulase with the activity function $A_1$(pH), can be designed to protect the entrapped material from the environment in the human stomach, and release the entrapped material in the human small intestine in any of the above considered cases if the material is designed with a cumulated activity specification in the window 17 mma to 165 mma.

**Example 2**

The model predicting the performance of the self-perforating material in the gastro-intestinal system $A_1(pH(t))/100$ was modified to study the performance of the self-perforating material in the human GI tract when the coating is given a time $T_{PM}$ post meal ingression. The formula for the pH variation then becomes:

$$pH(t) = \begin{cases} 0.5 + \exp(-0.016 * (T_{PM} + t) + 1.7), & 0 \leq t \leq T_S, \\ 6.1, & T_S \leq t \leq T_S + T_I. \end{cases}$$

The obtained model was used to study the cumulated activity when the coating is given at $T_{PM}$ = 60 min post meal ingression and considering a stomach transit time $T_S$ of 30 min. A graphic representation is shown in figure 11. Figure 4 shows the cumulated activity in the self-perforating material in the human gastro intestinal tract when the material is given at $T_{PM}$ = 60 min post meal ingression and considering a stomach transit time $T_S$ of 30 min and a small intestine transit time $T_S$ of 275 min.

When the coating is given 60 min post meal ingression the cellulase activity is very low because of the acidic environment. This is reflected in the predicted cumulated activity of 0 mma in the stomach. When the coating material enters the small intestine the cellulase activity increases, initiating the breakdown of the coating, and supporting the release of the entrapped material. By ingression of the coating at a delayed time following a meal, the cumulated activity in the stomach is decreased significantly compared to when the coating is ingressed together with a meal. Thus, the design space for the coating is increased significantly.

**Example 3**

The effects on the cumulated activity in the gastro intestinal tract of using cellulase enzymes with other pH optima was studied.

The model predicting the performance of the self-perforating material in the gastro-intestinal system was modified to $A_2(pH(t))/100$ to predict the performance in the human GI tract of a cellulose-cellulase self-perforating material, when the cellulase has maximal activity at $pH_{OPT}$. $A_2(pH)$ models a cellulase enzyme with characteristics similar to the cellulase properties described in model $A_1(pH)$, however, with an enzymatic optimum at $pH_{OPT}$.

$$A_2(pH) = 100 * exp\left(-\left(\frac{|pH - pH_{OPT}|}{2}\right)^3\right) \%ma$$

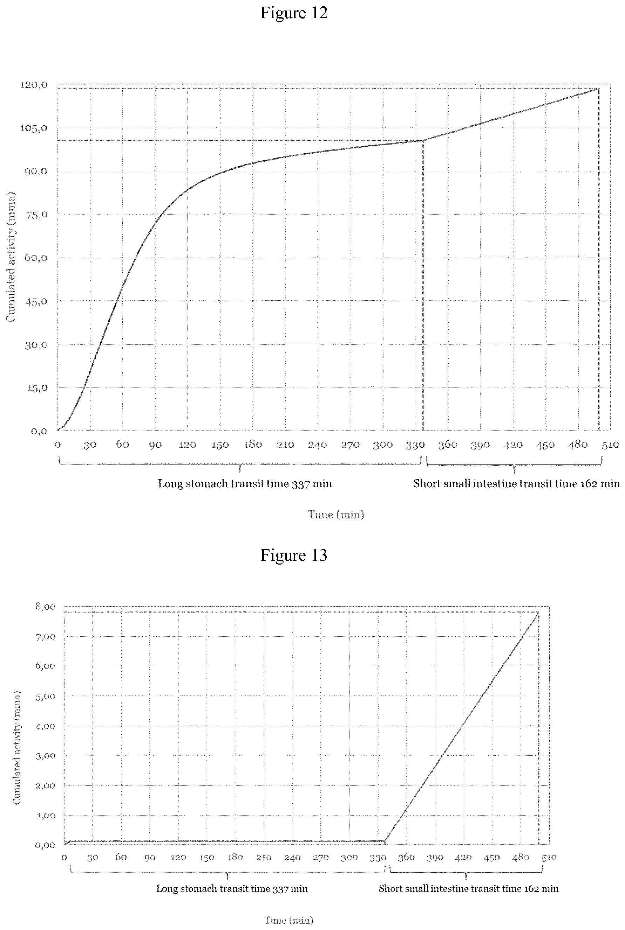

$A_2(pH)$ was used to study the cumulated activity of an enzyme with maximal activity at $pH_{OPT}$ equal to 3.5. A graphic representation is shown in figure 12. Figure 5 shows the graphic representation of the cumulated activity of an enzyme with maximal activity at $pH_{OPT}$ = 3.5 described in function $A_2(pH(t))/100$.

$A_2(pH)$ was used to study the cumulated activity of an enzyme with the maximal activity at $pH_{OPT}$ equal to 9.0. A graphic representation is shown in figure 13. Figure 6 shows the graphic representation of the cumulated activity of an enzyme with maximal activity at $pH_{OPT}$ = 9.0 described by function $A_2(pH(t))/100$.

In a composition with a cellulase with maximal activity at pH 3.5 the cumulated activity in the stomach levels out at almost zero at prolonged stomach transit times (figure 12). A composition with an enzyme with maximal activity at pH 3.5 can protect the entrapped material in the stomach when the coating material is designed to release the entrapped material in the range 100 mma to 118 mma. However, such a composition with an enzyme with maximal activity at pH 3.5 may require that the coating is given post meal ingression to obtain a robust design space for release of the entrapped material in the small intestine.

A composition with an enzyme with maximal activity at pH 9.0 can protect the entrapped material in the stomach when the coating material is designed to release the entrapped material in the range 0.1 mma to 7.8 mma. A very robust design space for releasing the entrapped material in the intestine can be obtained with such a composition.

A composition with an enzyme with maximal activity above pH 9.0 will only improve the ability of the material to protect the entrapped material in the stomach and release the entrapped material in the intestine.

A composition with a cellulase enzyme with an enzymatic optimum in the range from at least pH 3.0 to 12.0, such as from 3.5 to 9.0, can serve as the second component in the self-perforating material, and support the protection of the entrapped material in the stomach and a release in the small intestine.

**Conclusion**

Based on the studies presented the described coating material allows protection of the entrapped material in the stomach of a mammal, such as a human, and allows for release in the small intestine. Together example 1 to 3 show that a high degree of robustness of design space can be obtained, allowing for a wide range of coating specifications.

**References**

[1] Dressman JB, Berardi RR, Dermentzoglou LC, Russell TL, Jarvenpaa KM. Upper Gastrointestinal (GI) pH in Young, Healthy Men and Women. Pharm Res. 1990; 7:756-761.

[2] Clarysse S, Tack J, Lammert F, Duchateau G, Reppas C, Augustijns P. Postprandial Evolution in Composition and Characteristics of Human Duodenal Fluids in Different Nutritional States. J. Pharm Sci. 2009; 98:1177-1192.

[3] Zarate N, Mohammed SD, O'Shaughnessy E, Newell M, Yazaki E, Williams NS, Lunniss PJ, Semler JR, Scott SM. Am J Physiol - Gastroint and Liver Physiol 2010; 299 (6): G1276-G1286. Accurate localization of a fall in pH within the ileocecal region: Validation using a dual-scintigraphic technique.

[4] Worsøe et al. Gastric transit and small intestinal transit time and motility assessed by a magnet tracking system BMC Gastroenterology 2011, 11:145

[5] Assessment of regional gut transit times in healthy controls and patients with gastroparesis using wireless motility technology. I. Sarosiek et al. Aliment Pharmacol Ther. 2010 January 15; 31(2): 313-322

[6] Occurrence of cellulose, Kyle Ward, JR. In Cellulose and Cellulose Derivatives, Part I; Ott, E., Spurlin, H.M., Eds.; Interscience Publishers Inc. New York, 1954; Vol. V, p 9-29

[7] Ultrafine Cellulose Fibers Produced by Asaia bogorensis, an Acetic Acid Bacterium, A. Kumagai et al. Biomacromolecules 2011, 12, 2815-2821

[8] Sensing the structural differences in cellulose from apple and bacterial cell wall materials by Raman and FT-IR spectroscopy. Szymanska-Chargot M, Cybulska J, Zdunek A. Sensors (Basel). 2011;11(6):5543-60.

[9] BRENDA, the enzyme information system in 2011. Nucleic Acids Res. 2011, 39: D670-D676, www.brenda-enzymes.org.

[10] A novel efficient β-glucanase from a paddy soil microbial metagenome with versatile activities. Biotechnol Biofuels. 2016 Feb 13;9:36

[11] Screening and characterization of a cellulase with endocellulase and exo-cellulase activity from yak rumen metagenome J. Mol. Catal. B 73, 104-110 (2011)

[12] Cloning and Characterization of an Endoglucanase Gene from Actinomyces sp. Korean Native Goat 40. Asian Australas. J. Anim. Sci. Vol. 29, No. 1: 126-133 January 2016.

## Claims

1. A solid oral composition for targeted release in an intestine of a mammal comprising a core and a coating completely surrounding the core, wherein the core comprises an entrapped material to be released in the intestine, and the coating or a part of the coating is adapted to be digested in the intestine.

2. The composition of claim 1 wherein the coating is adapted to resist breakdown from the environment in a stomach of a mammal.

3. The composition of any one of the preceding claims wherein the coating prevents the release of the entrapped material in a stomach of a mammal.

4. The composition of any one of the preceding claims wherein the coating protects the entrapped material in a stomach of a mammal.

5. The composition of any one of the preceding claims wherein the coating is adapted to release the entrapped material in an intestine of a mammal.

6. The composition of any one of the preceding claims wherein the coating comprises a pair of components.

7. The composition of any one of the preceding claims wherein the first component is resistant to an environment in the stomach of a mammal and the second component digest the first component when subjected to the more basic environment of the intestine compared to the more acidic environment of the stomach.

8. The composition of any one of the preceding claims where the digesting activity of the second component is inhibited in the environment in the stomach of a mammal.

9. The composition of any one of the preceding claims wherein the mammal is selected from animals with little or no ability to digest cellulose, such as a human, a monkey, a pig, a dog, a human ape, a rodent and a cat.

10. The composition of any one of the preceding claims wherein the entrapped material is a medicinal product, such as a protein, an enzyme, a polypeptide, an oligopeptide, a peptide, a deoxyribonucleic acid (DNA), a ribonucleic acid (RNA), a small organic molecule of less than 900 Da, or a pro-drug of any one of these materials, is a nutritional supplement, or a microbial culture, or a microbial additive, or a vaccine.

11. The composition of any one of the preceding claims wherein the first component and the second component are mixed.

12. The composition of any one of the preceding claims wherein the first component and the second component are in different layers with the first component being the outer layer and the second component the inner layer around the core.

13. The composition of any one of the preceding claims wherein the pair of the first and second component is selected from cellulose and cellulase, pectin and pectinolytic enzymes, hemicellulose and hemicellulase, lignin and lignin degrading enzymes, fructan and fructan degrading enzymes, and lipid and lipases.

14. The composition of any one of the preceding claims wherein the pair of the first and second component is selected from structurally ordered cellulose I such as bacterial cellulose or derivatives thereof and a cellulase, such as cellulase (EC 3.2.1.4) (endocellulase) and cellubiase (EC 3.2.1.21) (beta-glucosidase) and 1,4-beta-cellobiosidase (EC 3.2.1.91) (Exocellulase).

15. The composition of any one of the preceding claims wherein the second component is enzymatically active and digests the first component in the environment in the intestine of a mammal.

16. The composition of claim 15 wherein the second component has maximum enzymatic activity in the range pH 3-12, such as pH 3.5 to 9.5, or 3.0 to 9.0, or 4.0 to 9.0.

17. The composition of any one of the preceding claims wherein the intestine is selected from small intestine, large intestine, duodenum, ileum, jejunum, and colon.

18. The composition of any one of the preceding claims wherein the composition is a tablet or a capsule or other type of solid oral dosage form.

19. The composition of any one of claims 1-18 for use as a medicinal product.

20. The composition of any one of claims 1-18 for use as a nutritional supplement.

21. The composition of any one of claims 1-18 for use as a tracing agent,

22. Use of a pair of components for preparing a coating for a solid oral dosage form where the coating is degraded when subjected to a pH change from a lower to a higher pH, wherein the digestion of the first component by the second component is inhibited at the lower pH and the second component digest the first component when subjected to the higher pH.

23. The composition of any one of claims 1-21, wherein the entrapped material is selected from:

   e) Proteins, Human growth hormone (hGH), Calcitonin, Insulin, GLP-1 analogues, GLP-1
   f) Peptides, Octreotide
   g) Oral vaccines, Oral cholerae vaccine, Mycoplasma hyopneumoniae oral vaccine, Live bacterial cells as attenuated vaccines, live cell culture.
   h) Small organic molecules 200<MW<900 g/mol, Desmopressin, Vasopressin, Cyclo-sporine, Ranitidine, Diclofenac, Ketoprofen, Amifostine, Omeprazole, Gemcitabine, Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Lobenzarit, Genistein, Verapamil, Terbinafine, Lornoxicam, Clotrimazole.

Figure 1

Small intestine

Esophagus | Stomach | Duodenum | Jejunum | Ileum | Colon

Transit time:
Seconds

pH 6-7

Digesting
enzymes

Transit time: minutes
to 5.5 hours

pH ≤ 6.0 at meal
pH ≤ 3.5 45 min after meal
pH ≤ 1.5 120 min after
meal

Gastric fluid:
Pepsin and other digesting
enzymes

Transit time: 2.5 – 11 hours

pH 5 – 7

Figure 2

Produce cellulase X
(CX) by microbial
fermentation

Affinity purify CX to
GMP grade

BC/CX coating with
specific minutes of
maximal activity (mma)
before coating perforation

Produce bacterial
cellulose (BC) cuticle
by microbial
fermentation

Purify BC cuticle to
GMP grade

Infuse CX into the wet
BC cuticle

Drying cuticle yielding
sheets of BC with CX
(BC/CX coating
sheets)

Embed tablet in the
BC/CX coating sheets
and seal

Key Process Parameters: | Cuticle height | Ratio CX(spec act)/BC (g) | | Seal efficiency

Figure 3

Figure 4

## Figure 5

Stomach transit time 127 min.  Small intestine transit time 162 min.

Time (min)

## Figure 6

Stomach transit
time 60 min

Small intestine transit time 275 min

Time (min)

Figure 7

Figure 8

Stomach transit time 337 min

Small intestine transit time 162 min

Time (min)

Figure 9

Stomach transit
time 5 min

Time (min)

Small intestine transit time 669 min

Figure 10

Stomach transit time 337 min          Small intestine transit time 669 min

Time (min)

Figure 11

60 min post meal          Small intestine transit time 275 min
ingression

Time (min)

Figure 12

Long stomach transit time 337 min    Short small intestine transit time 162 min

Time (min)

Figure 13

Long stomach transit time 337 min    Short small intestine transit time 162 min

Time (min)

Europäisches
Patentamt
European
Patent Office
Office européen
des brevets

# EUROPEAN SEARCH REPORT

**Application Number**

EP 18 18 8848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/199303 A1 (VRETTOS JOHN [US] ET AL) 14 July 2016 (2016-07-14) * the whole document * * paragraph [0203] - paragraph [0213]; claims 1-56 * | 1-15 | INV. A61K9/28 A61K9/48 |
| X | WO 2017/168426 A1 (VITAL BEVERAGES GLOBAL INC [GB]; BARZILAY AMIR [IL]) 5 October 2017 (2017-10-05) * the whole document * * paragraph [0208] - paragraph [0220]; claims 1-37 * | 1-15 | |
| X | WO 2008/150426 A1 (UNIGENE LAB INC [US]; STERN WILLIAM [US]) 11 December 2008 (2008-12-11) * the whole document * * claims 1-55 * | 1-15 | |

| | | | TECHNICAL FIELDS SEARCHED (IPC) |
|---|---|---|---|
| | | | A61K |

The present search report has been drawn up for all claims

| Place of search | Date of completion of the search | Examiner |
|---|---|---|
| Munich | 19 June 2019 | Felder, Christian |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&  : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 8848

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2019

| Patent document cited in search report | | Publication date | Patent family member(s) | | Publication date |
|---|---|---|---|---|---|
| US 2016199303 | A1 | 14-07-2016 | AU | 2016206950 A1 | 27-07-2017 |
| | | | CA | 2973099 A1 | 21-07-2016 |
| | | | CN | 107427469 A | 01-12-2017 |
| | | | EP | 3244878 A1 | 22-11-2017 |
| | | | JP | 2018501292 A | 18-01-2018 |
| | | | KR | 20170103942 A | 13-09-2017 |
| | | | US | 2016199303 A1 | 14-07-2016 |
| | | | US | 2018049987 A1 | 22-02-2018 |
| | | | WO | 2016115082 A1 | 21-07-2016 |
| WO 2017168426 | A1 | 05-10-2017 | CA | 3019745 A1 | 05-10-2017 |
| | | | CN | 109562070 A | 02-04-2019 |
| | | | EP | 3435983 A1 | 06-02-2019 |
| | | | JP | 2019513146 A | 23-05-2019 |
| | | | KR | 20190026648 A | 13-03-2019 |
| | | | WO | 2017168426 A1 | 05-10-2017 |
| WO 2008150426 | A1 | 11-12-2008 | AU | 2008260615 A1 | 11-12-2008 |
| | | | CA | 2689358 A1 | 11-12-2008 |
| | | | CN | 101801309 A | 11-08-2010 |
| | | | DK | 2152195 T3 | 15-02-2016 |
| | | | EP | 2152195 A1 | 17-02-2010 |
| | | | EP | 3009129 A1 | 20-04-2016 |
| | | | ES | 2560251 T3 | 18-02-2016 |
| | | | JP | 5365629 B2 | 11-12-2013 |
| | | | JP | 2010529018 A | 26-08-2010 |
| | | | PT | 2152195 E | 08-02-2016 |
| | | | US | 2009317462 A1 | 24-12-2009 |
| | | | US | 2012040000 A1 | 16-02-2012 |
| | | | US | 2012315325 A1 | 13-12-2012 |
| | | | US | 2013034600 A1 | 07-02-2013 |
| | | | US | 2014335169 A1 | 13-11-2014 |
| | | | US | 2016339081 A1 | 24-11-2016 |
| | | | WO | 2008150426 A1 | 11-12-2008 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DRESSMAN JB ; BERARDI RR ; DERMENT-ZOGLOU LC ; RUSSELL TL ; JARVENPAA KM.** Upper Gastrointestinal (GI) pH in Young, Healthy Men and Women. *Pharm Res.,* 1990, vol. 7, 756-761
- **CLARYSSE S ; TACK J ; LAMMERT F ; DUCHATEAU G ; REPPAS C ; AUGUSTIJNS P.** Postprandial Evolution in Composition and Characteristics of Human Duodenal Fluids in Different Nutritional States. *J. Pharm Sci.,* 2009, vol. 98, 1177-1192
- **ZARATE N ; MOHAMMED SD ; O'SHAUGHNESSY E ; NEWELL M ; YAZAKI E ; WILLIAMS NS ; LUNNISS PJ ; SEMLER JR ; SCOTT SM.** *Am J Physiol - Gastroint and Liver Physiol,* 2010, vol. 299 (6), G1276-G1286
- **WORSØE et al.** Gastric transit and small intestinal transit time and motility assessed by a magnet tracking system. *BMC Gastroenterology,* 2011, vol. 11, 145
- **I. SAROSIEK et al.** Assessment of regional gut transit times in healthy controls and patients with gastroparesis using wireless motility technology. *Aliment Pharmacol Ther.,* 15 January 2010, vol. 31 (2), 313-322
- In Cellulose and Cellulose Derivatives. **KYLE WARD, JR.** Occurrence of cellulose. Interscience Publishers Inc, 1954, vol. V, 9-29
- **A. KUMAGAI et al.** Ultrafine Cellulose Fibers Produced by Asaia bogorensis, an Acetic Acid Bacterium. *Biomacromolecules,* 2011, vol. 12, 2815-2821
- **SZYMAŃSKA-CHARGOT M ; CYBULSKA J ; ZDUNEK A.** Sensing the structural differences in cellulose from apple and bacterial cell wall materials by Raman and FT-IR spectroscopy. *Sensors (Basel),* 2011, vol. 11 (6), 5543-60
- **BRENDA.** the enzyme information system in 2011. *Nucleic Acids Res.,* 2011, vol. 39, D670-D676, www.brenda-enzymes.org.
- A novel efficient β-glucanase from a paddy soil microbial metagenome with versatile activities. *Biotechnol Biofuels,* 13 February 2016, vol. 9, 36
- Screening and characterization of a cellulase with endocellulase and exo-cellulase activity from yak rumen metagenome. *J. Mol. Catal. B,* 2011, vol. 73, 104-110
- Cloning and Characterization of an Endoglucanase Gene from Actinomyces sp. Korean Native Goat 40. Asian Australas. *J. Anim. Sci.,* January 2016, vol. 29 (1), 126-133